# EUROPEAN PATENT APPLICATION

(11) **EP 1 992 292 A1**
(43) Date of publication of application: **19.11.2008**
(21) Application number: 07737608.5
(22) Date of filing: 01.03.2007
(51) Int. Cl.: A61B 8/12

(54) **ULTRASONIC ENDOSCOPE**

(30) Priority: 03.03.2006 JP 2006058708
(71) Applicant: Olympus Medical Systems Corp., Tokyo 151-0072 (JP)
(72) Inventor: KODAMA, Hiroshi, Tokyo 151-0072 (JP); NAKAZATO, Takeharu, Tokyo 1051-0072 (JP)
(74) Representative: von Hellfeld, Axel
(86) International application number: PCT/JP2007/053928
(87) International publication number: WO 2007/100050

(57) **Abstract**

An ultrasound endoscope 1 including, at a distal end of an insertion portion 2 to be inserted to a body cavity, a distal rigid portion 2a arranged forward of a flexible tube portion 2c, an ultrasound transducer portion 10 for scanning a plane which is parallel to forward side of a longitudinal center axis L1 of the distal rigid portion 2a, and a treatment instrument insertion channel port 27 opening in a distal-side end face of the distal rigid portion 2a, with a longitudinal center axis L4 being parallel to the longitudinal center axis L1 of the distal rigid portion.

## Description

### Technical Field

The present invention relates to an ultrasound endoscope having an observation optical system, an instrument channel, and a convex-type ultrasound transducer, which are provided at the distal end portion of an insertion portion.

### Background Art

Ultrasound endoscopes that have been known to conduct ultrasound diagnosis of a body cavity interior include one having a convex-type ultrasound transducer. Such a convex-type ultrasound transducer is configured by arranging a plurality of transducer arrays in a projected circular-arc form.

As an ultrasound endoscope having such a convex-type ultrasound transducer, there is an ultrasound endoscope disclosed in Japanese Patent Application Laid-Open Publication No. 8-131442, for example. The ultrasound endoscope is provided, at its distal rigid portion, with an ultrasound transducer which gives a forward oblique view of an ultrasound scanning region, in addition to an observation optical system.

However, the ultrasound endoscope of Japanese Patent Application Laid-Open Publication No. 8-131442 is configured to project a treatment instrument toward the ultrasound scanning region which is given as a forward oblique view. That is, the treatment instrument is obliquely led out of the insertion portion with respect to the insertion direction.

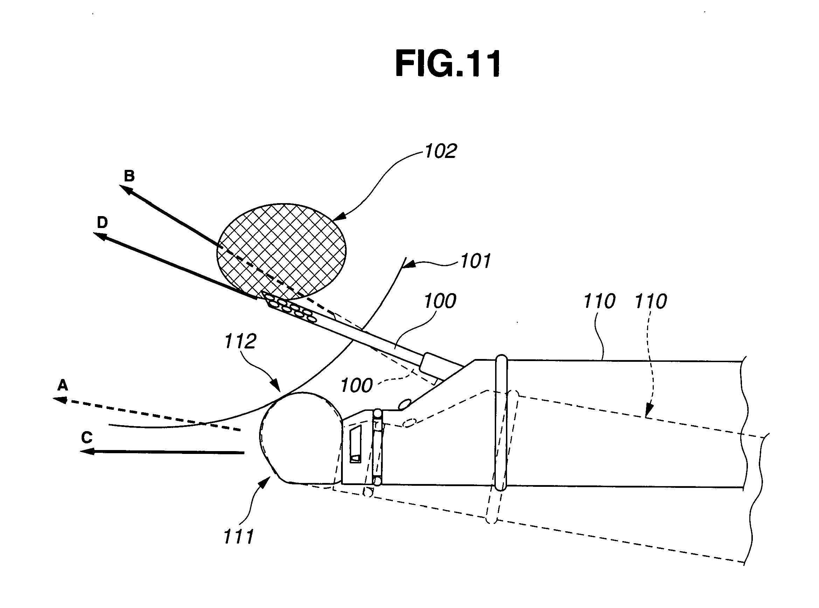

Thus, as shown in Fig. 11, when a treatment instrument 100 is led out toward a treatment region 102 in a stomach through a stomach wall 101, the operator first tilts an endoscope insertion portion 110 as indicated by a broken line and, in this state, brings a transducer lens surface 112 of an ultrasound transducer 111 into close contact with the stomach wall 101. Then, while keeping the close-contact state, the operator manipulates the treatment instrument 100 so as to be led in toward the treatment region 102. In this case, the insertion direction, i.e. the pressing direction, of the endoscope insertion portion 110 will be as indicated by a broken arrow A, and the lead-out direction of the treatment instrument 100 will be as indicated by a broken arrow B.

Accordingly, when the treatment instrument 100 is projected from the endoscope insertion portion 110, the endoscope insertion portion 110 may be displaced as indicated by the solid line because the lead-out direction and the insertion direction are not identical. Then, the insertion direction of the endoscope insertion portion 110 may probably be changed in the direction indicated by a solid arrow C, resulting in a possible change of the lead-out direction of the treatment instrument 100 in the direction indicated by a solid arrow D. In this case, the lead-out direction of the treatment instrument 100 is deviated from the treatment region 102, bringing difficulty in conducting desired treatment.

Meanwhile, in the ultrasound endoscope of Japanese Patent Application Laid-Open Publication No. 8-131442, the ultrasound transducer is projected to the distal end side of the distal rigid portion having the observation optical system or the like. Accordingly, such drawbacks have probably been caused during optical observation, as partial blocking of an observation field of view by the ultrasound transducer, or blocking of observation due to the shadow of the ultrasound transducer portion, which shadow is produced in the optical image. Also, in the ultrasound observation, such a drawback may have been caused as reflection of the ultrasound by an air/water supply nozzle, for example, to produce an artifact in the ultrasound image and to thereby block the observation. For this reason, there has been a demand for a design which is able to avoid these drawbacks of blocking observation.

The present invention has been made in light of the circumstances as described above, and has an object of providing an ultrasound endoscope which enables forward optical observation along the insertion direction of the insertion portion, and can reliably lead out a treatment instrument toward a target region of a deep portion forward along the insertion direction, which target region is visualized in the ultrasound image.

In addition, the present invention has another object of providing an ultrasound endoscope which well enables observation through an observation optical system, as well as observation through ultrasound image obtained by an ultrasound transducer portion.

### Disclosure of Invention

### Means for Solving the Problem

An ultrasound endoscope of the present invention has an insertion portion to be inserted into a body cavity, the insertion portion comprising at a distal end thereof: a distal rigid portion arranged forward of a flexible tube portion; an ultrasound transducer portion for scanning a plane which is parallel to forward side of a longitudinal center axis of the distal rigid portion; and a treatment instrument insertion channel port opening in a distal-side end face of the distal rigid portion, the port having a longitudinal center axis which is parallel to the longitudinal center axis of the distal rigid portion.

The ultrasound endoscope of the present invention has a distal rigid portion configuring an insertion portion to be inserted into a body cavity and arranged forward of a flexible tube portion, the ultrasound endoscope comprising at the distal rigid portion thereof: an ultrasound transducer portion for scanning a plane which is parallel to forward side of a longitudinal center axis of the distal rigid portion; and a treatment instrument insertion channel port configuring an instrument channel for leading out a treatment instrument with respect to a scanning plane forward of the ultrasound transducer portion, the port having a longitudinal center axis which is parallel to the longitudinal center axis of the distal rigid portion, wherein:
a distal end surface of a distal rigid portion comprises an observation window configuring an observation optical system, an illumination window configuring an illumination optical system, and an air/water supply nozzle for ejecting fluid at least to a surface of the observation window; the air/water supply nozzle is arranged outside an ultrasound observation region possessed by the ultrasound transducer portion; the observation window is arranged at a position outside the ultrasound observation region possessed by the ultrasound transducer portion, the position corresponding to a position where the ultrasound transducer portion is permitted to fall outside an observation field of view of the observation optical system; and the illumination window is arranged at a position outside the ultrasound observation region possessed by the ultrasound transducer portion, the position corresponding to a position on a more outer peripheral side than the observation window.

### Brief Description of the Drawings

Fig. 1 is an illustration explaining a configuration of an ultrasound endoscope;
Fig. 2 is a perspective view illustrating a distal end portion of an ultrasound endoscope;
Fig. 3 is a front elevational view of the distal end portion illustrated in Fig. 2, as viewed from the front;
Fig. 4 is a top view of the distal end portion illustrated in Fig. 2, as viewed from the top;
Fig. 5 is a cross-sectional view taken along a line A-A of Fig. 3;
Fig. 6 is an illustration explaining a relationship between: an ultrasound transducer configured by arranging a plurality of piezoelectric elements; and an ultrasound observation region of the ultrasound transducer and a treatment instrument led out from an instrument channel;
Fig. 7 is an illustration indicating an example of an ultrasound image including an artifact as appeared;
Fig. 8 is an illustration indicating an example of an ultrasound image visualized by the ultrasound transducer illustrated in Fig. 6;
Fig. 9 is an illustration explaining a relationship between a tissue contact surface of a nose piece and a transducer lens surface of an ultrasound transducer;
Fig. 10 is an illustration explaining an operation of an ultrasound endoscope; and
Fig. 11 is an illustration explaining a state where a treatment instrument is led into a treatment region through a stomach wall, using a conventional ultrasound endoscope.

### Best Mode for Carrying Out the Invention

With reference to the drawings, an embodiment of the present invention will hereinafter be described in detail.

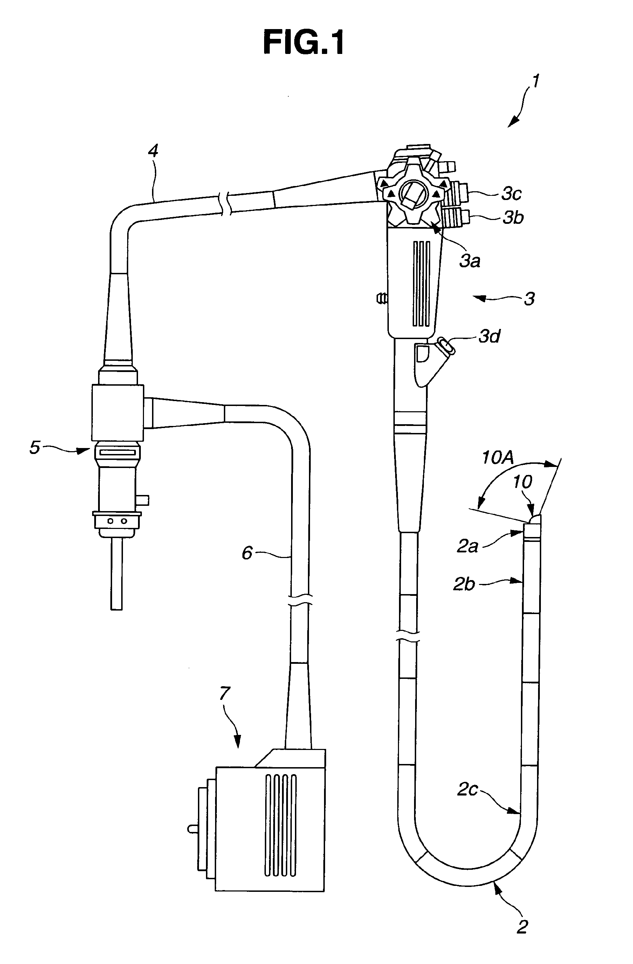

With reference to Figs. 1 to 10, the embodiment of the present invention will be described in detail.

As shown in Fig. 1, an ultrasound endoscope 1 of the present embodiment is configured by being provided with an elongated insertion portion 2 to be inserted into a body cavity, an operation portion 3 provided at a proximal end of the insertion portion 2, and a universal cord 4 extending from a side portion of the operation portion 3. An endoscope connector 5 is provided at the proximal end portion of the universal cord 4. An ultrasound cable 6 is extended from a side portion of the endoscope connector 5. An ultrasound connector 7 is provided at the proximal end portion of the ultrasound cable 6.

The insertion portion 2 is configured by sequentially connecting, from the distal end side, a distal rigid portion 2a formed of a rigid member, a bending portion 2b configured so as to be bendable, and an elongated flexible tube portion 2c having flexibility and extending from the proximal end of the bending portion 2b to the distal end of the operation portion 3. Indicated by reference 10 is an ultrasound transducer portion provided with a convex-type ultrasound transducer that will be described later. The ultrasound transducer 10 forms an ultrasound observation region 10A for scanning in a forward direction along an insertion axial direction. In other words, the ultrasound transducer portion 10 has the ultrasound observation region 10A for scanning in a forward direction.

The operation portion 3 is provided with an angle knob 3a for performing bending operation. The operation portion 3 is also provided with an air/water supply button 3b for performing operation of air/water supply and a suction button 3c for performing suction. The operation portion 3 is further provided with a treatment instrument insertion port 3d for introducing a treatment instrument into a body cavity.

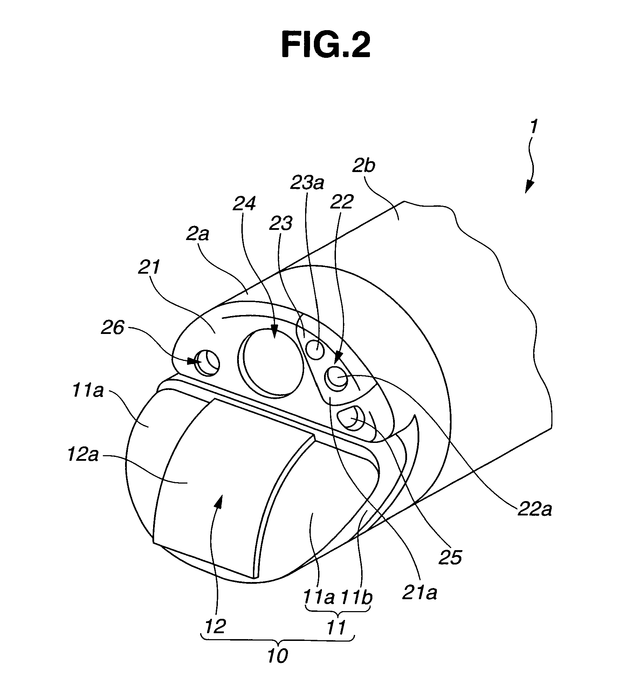

As shown in Fig. 2, the distal rigid portion 2a of the insertion portion 2 is provided with the ultrasound transducer portion 10 for obtaining ultrasound acoustic image information. The ultrasound transducer portion 10 is configured by being provided with a nose piece 11 serving as a housing and an ultrasound transducer 12. The ultrasound transducer 12 is arranged for integration at a notch portion which is formed substantially at the center of the nose piece 11.

A tissue contact surface 11a and a transducer lens surface 12a of the ultrasound transducer 12, which configure the nose piece 11, are configured being projected from a distal end surface 21 of the distal rigid portion 2a.

The distal end surface 21 of the distal rigid portion 2a is provided with an observation window 22a configuring an observation optical system 22, an illumination window 23a configuring an illumination optical system 23, an instrument channel (hereinafter shortened as a lead-out port) 24 for leading out a treatment instrument, such as a puncture needle, an air/water supply nozzle 25 for ejecting fluid, such as water and air, toward the observation window 22a, and an auxiliary water supply channel port 26 for supplying water in the forward direction.

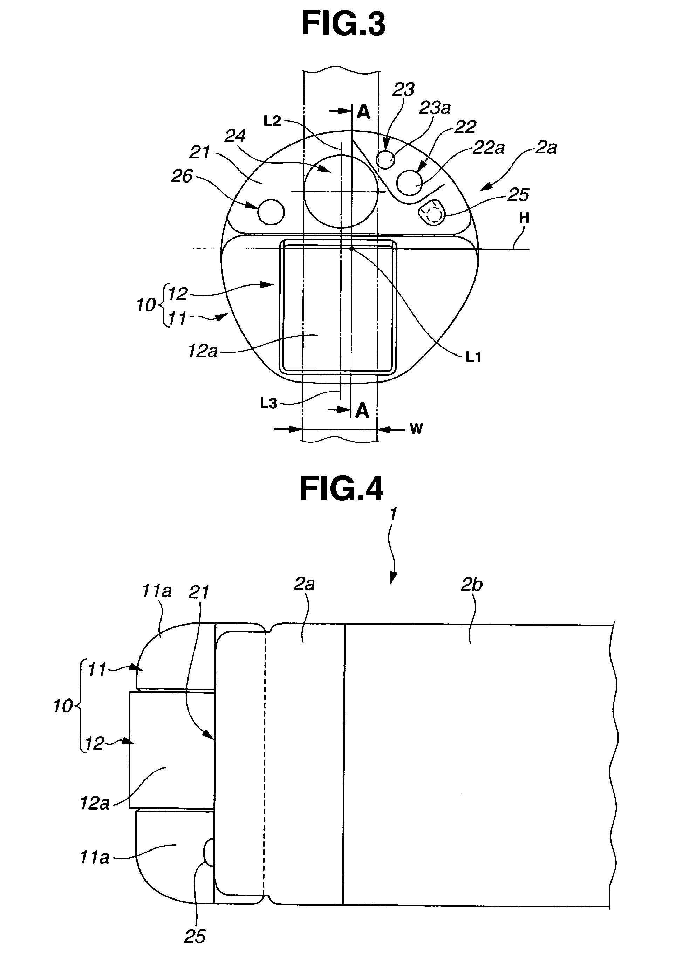

As shown in Fig. 3, in the ultrasound endoscope 1 according to the present embodiment, the distal end surface 21 is divided into a section for endoscopic observation, which is the upper side of a horizontal line H passing a longitudinal center axis L1 of the distal rigid portion 2a, and a section for ultrasound observation, which is the lower side.

It is so configured that a vertical center line L2 of the lead-out port 24 is substantially aligned with a vertical center line L3 of the transducer lens surface 12a of the ultrasound transducer 12.

The radial dimension of the lead-out port 24 is formed to have a size that falls within a width dimension W of an ultrasound observation region 10A formed by the ultrasound emitted from the transducer lens surface 12a, which area is indicated by a dash-dot-dot line. As a result, a treatment instrument led out from the lead-out port 24 reliably moves within the ultrasound observation region 10A.

The observation window 22a, the illumination window 23a and the air/water supply nozzle 25 are collectively arranged on one side which corresponds, for example, to the right side of the figure, with respect to the lead-out port 24. Also, the observation window 22a, the illumination window 23a and the air/water supply nozzle 25 are arranged outside the ultrasound observation region 10A.

Among the observation window 22a, the illumination window 23a and the air/water supply nozzle 25, the arrangement position of the air/water supply nozzle 25 is set so as to be located farthest from the ultrasound observation region 10A. This is because, as shown in Fig. 4, the air/water supply nozzle 25 is provided, being projected with respect to the distal end surface 21 of the distal rigid portion 2a. Specifically, the arrangement of the projected air/water supply nozzle 25 close to the ultrasound observation region 10A may cause the ultrasound emitted from the transducer lens surface 12a to be reflected by the air/water supply nozzle 25 to visualize the air/water supply nozzle in the ultrasound image. The farthest positioning is purposed for preventing such visualization.

In the present embodiment, the illumination window 23a, the observation window 22a and the air/water supply nozzle 25 are positioned so as to be aligned with each other, considering the objects of improving observation performance, of improving cleaning performance and of reducing the outer radial dimension of the distal end portion of the endoscope.

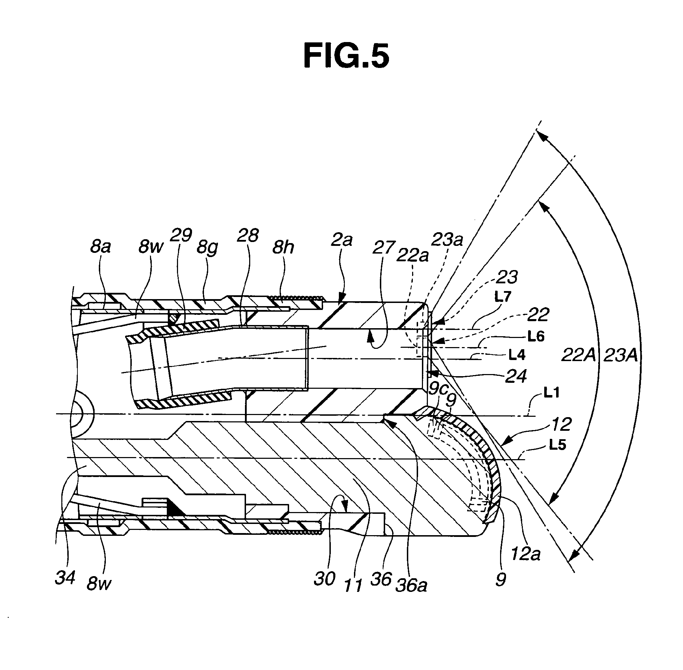

The observation window 22a is positioned being distanced from the ultrasound transducer portion 10, that is, positioned at an upper position in the figure, considering the observation field of view of the observation optical system 22, which will be described later, refer to the range of reference 22A indicated by a dash-dot line in Fig. 5. This may eliminate the drawback, i.e. partial lack of an endoscopic image displayed on a screen of a display device, not shown, which drawback would be caused by the blocking of the observation field of view by the ultrasound transducer portion 10 during the endoscopic observation.

On the other hand, the illumination window 23a is positioned being distanced from the ultrasound transducer portion 10, that is, positioned at an upper position on a more outer peripheral side than the observation window 22a, considering the radiation range of the illumination light from the illumination optical system 23, refer to the range of reference 23A indicated by a dash-dot-dot line in Fig. 5. This may eliminate the drawback, i.e. visualization of the ultrasound transducer portion 10 in the observation image during the endoscopic observation.

The observation window 22a and the illumination window 23a are provided in an observation distal end surface 21a which is configured to slightly project from the distal end surface 21. The auxiliary water supply channel port 26 is located on the other side, or the opposite side, of the side on which the observation window 22a, the illumination window 23a and the air/water supply nozzle 25 are arranged, the other side being the outside of the ultrasound observation region 10A.

As shown in Fig. 5, a distal bending piece 8a configuring the bending portion 2b is connected and fixed to the proximal end side of the distal rigid portion 2a. A plurality of bending pieces are sequentially connected to the distal bending piece 8a. Distal end portions of respective up-and-down and left-and-right bending wires 8w are fixedly set at predetermined positions of the distal bending piece 8a. Thus, with an operator's appropriate manipulation of the angle knob 3a, the relevant bending wires 8w are adapted to be tugged and released in response to the manipulation, so that bending motion can be imparted to the bending portion 2b can be bent.

The plurality of bending pieces are covered with bending rubber 8g. The distal end portion of the bending rubber 8g is integrally fixed to the distal rigid portion 2a by a bobbin bonded portion 8h.

The distal end surface 21 of the distal rigid portion 2a and the observation distal end surface 21a are configured so as to be orthogonal to the longitudinal center axis L1 of the distal rigid portion 2a. The distal rigid portion 2a is formed with a treatment instrument insertion channel port (hereinafter shortened as treatment instrument hole) 27 configuring the instrument channel 24, and also formed with an arrangement hole 30.

In addition to the holes 27 and 30, the distal rigid portion 2a is also provided, although not shown, with a through hole for setting the observation optical system, a through hole for setting the illumination optical system, an air/water supply through hole for supplying the fluid ejected from the air/water supply nozzle 25, a through hole configuring the auxiliary water supply channel port 26, and the like.

A longitudinal center axis L4 of the treatment instrument hole 27 is formed to be substantially parallel to the longitudinal center axis L1 of the distal rigid portion 2a. A longitudinal center axis L5 of the arrangement hole 30 is formed to be substantially parallel to the longitudinal center axis L1 of the distal rigid portion 2a. Optical axes L6 and L7 of the observation optical system and the illumination optical system, respectively, provided in the ultrasound endoscope 1 are also parallel to the longitudinal center axis L1 of the distal rigid portion 2a. Accordingly, the observation optical system provided in the ultrasound endoscope 1 of the present embodiment is of a so-called forward viewing type, in which the observation field of view is set at a forward front, that is, the observation field of view is set in the insertion direction, or is set forward along the longitudinal center axis L1 of the distal rigid portion 2a.

One end portion of a tube linking pipe 28 which is formed being inclined by a predetermined amount, is permitted to communicate with the proximal end side of the treatment instrument hole 27. One end portion of a channel tube 29 configuring a channel for inserting treatment instrument is permitted to communicate with the other end portion of the tube linking pipe 28. The other end portion of the channel tube 29 is permitted to communicate with the treatment instrument insertion port 3d.

Thus, a treatment instrument inserted through the treatment instrument insertion port 3d is smoothly moved through the channel tube 29, the tube linking pipe 28 and the treatment instrument hole 27, so as to be led out of the instrument channel 24 to the outside. The treatment instrument that has been led out of the instrument channel 24 is projected forward, or projected along the insertion direction for the insertion portion, being substantially parallel to the longitudinal center axis L1 of the distal rigid portion 2a.

Specifically, let us assume that the distal end portion of a puncture needle, as a treatment instrument, for example, is located in the treatment instrument hole 27. In this state, upon projection of a needle tube configuring the puncture needle, the needle tube is projected from the instrument channel 24 toward the forward front which is under observation through the observation window 22a, while being substantially parallel to the longitudinal center axis L1 of the distal rigid portion 2a.

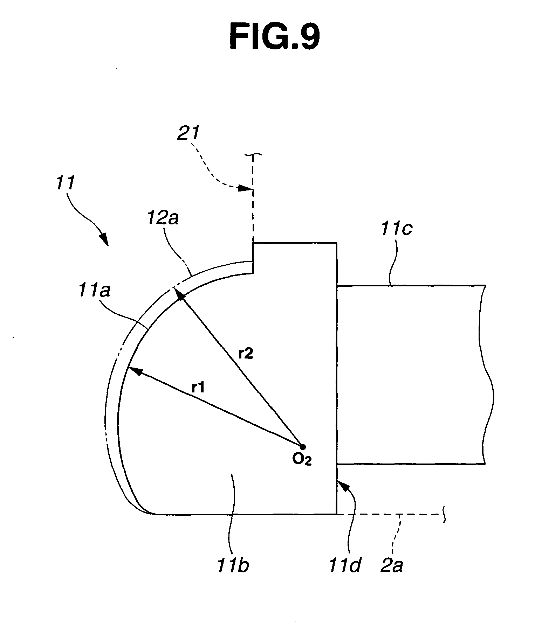

A fixing portion of the nose piece 11 is arranged in the arrangement hole 30, refer to reference 11c of Fig. 9 which will be described later. A distal end portion of an insulating tube, not shown, is fixedly permitted to communicate with the proximal end portion of the fixing portion 11c. An ultrasound cable 34 is inserted through the insulating tube. The ultrasound cable 34 is a bunch of a plurality of signal lines extended from a plurality of respective piezoelectric elements configuring the ultrasound transducer 12. The insulating tube extends through the insertion portion 2 so that the other end portion of the insulating tube reaches the operation portion 3. The ultrasound cable 34 extends through the insertion portion 2, the operation portion 3, the universal cord 4, the endoscope connector 5 and the ultrasound cable 6 to reach the ultrasound connector 7.

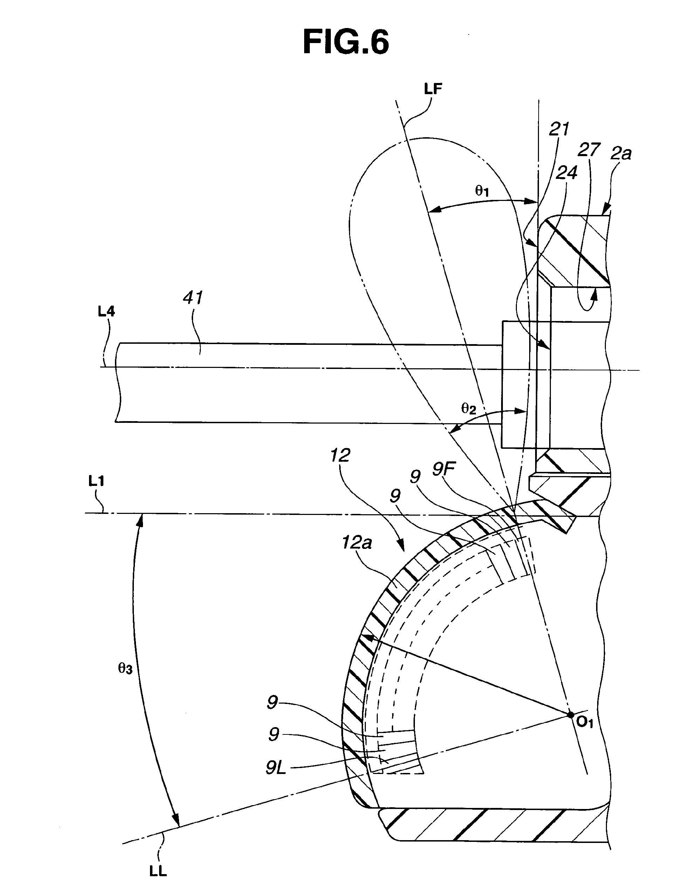

As shown in Figs. 5 and 6, the ultrasound transducer 12 of the ultrasound transducer portion 10 is provided at the center portion of the tissue contact portion 11b of the nose piece 11. The ultrasound transducer 12 is configured, for example, by a plurality of piezoelectric elements 9 and the transducer lens surface 12a. The plurality of piezoelectric elements 9 are arranged to form a projected arc.

As shown in Fig. 6, it is so configured that a center O1 of the arc forming the arc of the ultrasound transducer 12 which is configured by arranging the plurality of piezoelectric elements 9, is positioned on the side more proximal than the distal end surface 21 of the distal rigid portion 2a. The plurality of piezoelectric elements 9 having an arc form are arranged at a predetermined pitch from a first piezoelectric element 9F located on one end side, for emitting ultrasound to the vicinity of a projection-starting side, to a last piezoelectric element 9L located on the other end side.

The direction of a beam axis LF of the first piezoelectric element 9F is set so as to incline toward the distal end side by an angle θ1, with respect to the distal end surface 21 of the distal rigid portion 2a, in particular, with respect to the distal end surface 21 provided with the lead-out port 24.

In setting the direction of the beam axis LF of the first piezoelectric element 9F, being inclined by the angle θ1, a beam spread angle θ2 of the first piezoelectric element 9F is taken into consideration. Specifically, the angle θ1 is set so that at least a portion of the distal rigid portion 2a or at least a portion of the air/water supply nozzle 25, which is made of a material, such as metal or rigid resin, that can reflect ultrasound, for example, will not come into the beam spread angle enclosed by the dash-dot-dot line in the figure. The angle θ1 is set so as to at least exceed θ/2, i.e. one half of the beam spread angle θ2.

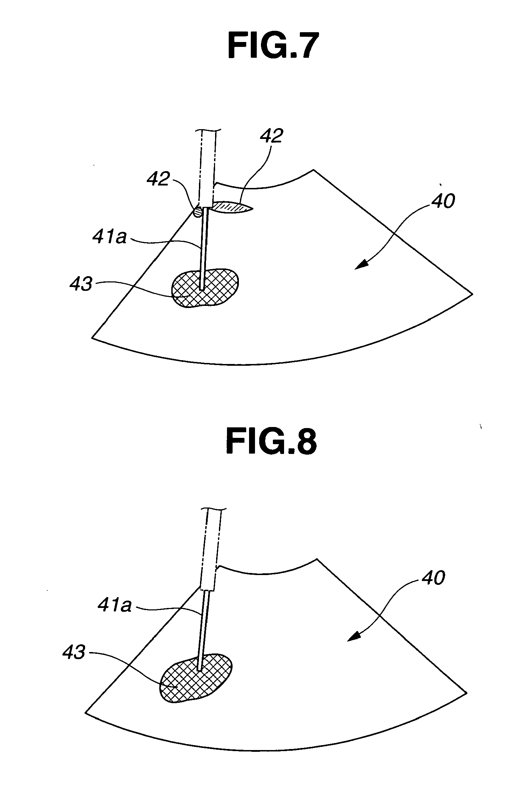

If the distal rigid portion 2a is present within the beam spread angle, an artifact 42 as shown in Fig. 7 appears. However, according to the configuration of the present application, no artifact appears, and a treatment instrument image 41 a is clearly visualized in an ultrasound image 40, as shown in Fig. 8. Thus, a treatment instrument 41 can be precisely led toward a lesion portion 43.

On the other hand, the direction of a beam axis LL of the last piezoelectric element 9L is set so as to be parallel to the longitudinal center axis L1 of the distal rigid portion 2a, or to be widened toward forward by an angle θ3.

As a result of such setting, when the treatment instrument 41 projected from the instrument channel 24 is projected forward substantially parallel to the longitudinal center axis L 1 of the distal rigid portion 2a, the treatment instrument 41 keeps moving within the ultrasound observation region 10A. Accordingly, as shown in Fig. 8, the treatment instrument image 41a is clearly visualized in the ultrasound image 40 from the state where the treatment instrument 41 is slightly projected from the instrument channel 24 to the point when the treatment instrument 41 punctures the lesion portion 43.

As shown in Figs. 2, 4, 5 and 9, the nose piece 11 has the tissue contact portion 11b and the fixing portion 11c. The tissue contact portion 11b has the tissue contact surface 11a of an arc form. The fixing portion 11c is arranged in the arrangement hole 30. The proximal end surface of the tissue contact portion 11b is a butting surface 11d, which is arranged being in contact with a planar surface portion 36a of a step portion 36. The planar surface portion 36a is provided with an opening, not shown, of the arrangement hole 30.

The outer radial dimension of a portion extending from the side of the butting surface 11d to the distal end of the nose piece 11 is set so as to have substantially the same dimension as the distal outer radial dimension of the distal rigid portion 2a, as shown in Figs. 2 to 4. Thus, the rigidity can be enhanced in the tissue contact portion 11b which is provided with the tissue contact surface 11a. In other words, the strength of the tissue contact portion 11b of the nose piece 11 is increased to achieve steady butting.

As shown in Fig. 9, the dimension of an arc radius r1 of the tissue contact surface 11a provided at the nose piece 11 is set to be the same as or slightly smaller than an arc radius r2 of the transducer lens surface 12a configuring the ultrasound transducer 12 and indicated by the dash-dot-dot line. A center O2 of the arc of the tissue contact surface 11a and the center O1 of the arc of the transducer lens surface 12a are set to be positioned on an axis parallel to the horizontal line H.

As described above, the transducer lens surface 12a of the ultrasound transducer 12 and the tissue contact surface 11a of the tissue contact portion 11b which is provided being interposed by the ultrasound transducer 12, are configured to substantially coincide with each other.

With such a configuration, when the ultrasound transducer portion 10 is pressed against body tissues for ultrasound observation, the tissue contact surface 11a and the transducer lens surface 12a are substantially uniformly brought into close contact with the body tissues. Thus, the ultrasound transducer portion 10 can be steadily pressed against the body tissues to obtain an ultrasound observation image.

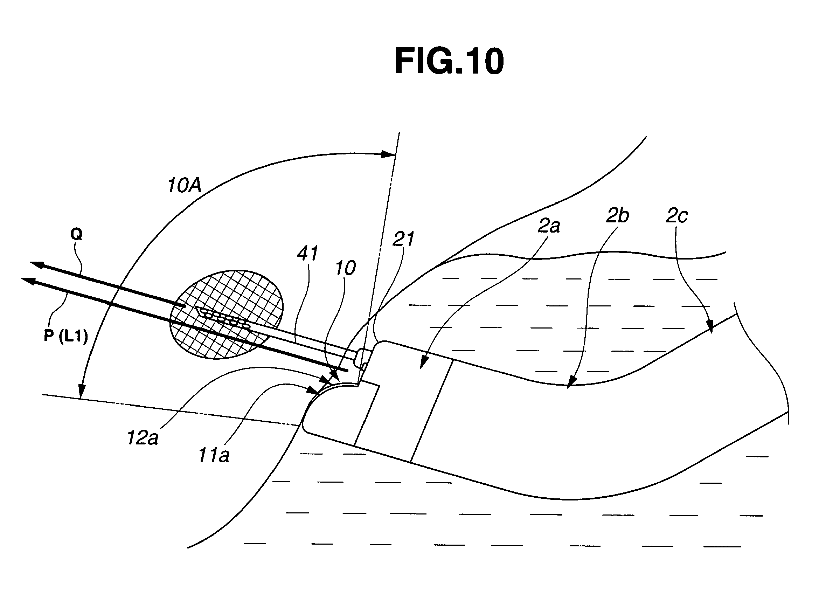

Also, in performing puncture using a treatment instrument under ultrasound observation, coincidence is attained between: the insertion direction of the insertion portion 2, which substantially goes along with the longitudinal center axis L1 of the distal rigid portion 2a and is indicated by an arrow P; and the puncture direction of the treatment instrument 41, which is indicated by an arrow Q, as shown in Fig. 10. As a result, upon operator's lead-in manipulation of the treatment instrument 41, a force associated with the treatment instrument 41 can be efficiently converted into a lead-in force to enhance the lead-in properties and the puncture properties of the treatment instrument 41.

It should be appreciated that the present invention is not limited only to the embodiment described above, but various modifications may be available within a scope not departing from the spirit of the invention.

The present application is filed claiming priority from Japanese Patent Application No. 2006-58708 filed in Japan on March 3, 2006. The content of disclosure of the above incorporated in the specification, claims and drawings of the present application.

## Claims

1. An ultrasound endoscope having an insertion portion to be inserted into a body cavity, the insertion portion comprising at a distal end thereof:
a distal rigid portion arranged forward of a flexible tube portion;
an ultrasound transducer portion for scanning a plane which is parallel to forward side of a longitudinal center axis of the distal rigid portion; and
a treatment instrument insertion channel port opening in a distal-side end face of the distal rigid portion, the port having a longitudinal center axis which is parallel to the longitudinal center axis of the distal rigid portion.

2. The ultrasound endoscope according to claim 1, wherein
the distal end of the insertion portion further comprises an observation optical system having an optical axis which is parallel to the longitudinal center axis of the distal rigid portion.

3. The ultrasound endoscope according to claim 1, wherein
the ultrasound transducer portion comprises a nose piece serving as a housing and an ultrasound transducer in which piezoelectric elements are arranged; and
the nose piece comprises: a tissue contact portion which is projected from the distal end surface of the distal rigid portion and has a tissue contact surface configured to coincide with the transducer lens surface; and a fixing portion which is fixed to the distal rigid portion.

4. The ultrasound endoscope according to claim 1, wherein
the nose piece has as fixing portion whose outer radial dimension on a proximal end side is set so as to be approximate to a distal-end outer radius of the distal rigid portion.

5. An ultrasound endoscope having a distal rigid portion configuring an insertion portion to be inserted into a body cavity and arranged forward of a flexible tube portion, the ultrasound endoscope comprising at the distal rigid portion thereof: an ultrasound transducer portion for scanning a plane which is parallel to forward side of a longitudinal center axis of the distal rigid portion; and a treatment instrument insertion channel port configuring an instrument channel for leading out a treatment instrument with respect to a scanning plane forward of the ultrasound transducer portion, the port having a longitudinal center axis which is parallel to the longitudinal center axis of the distal rigid portion, wherein
a distal end surface of a distal rigid portion comprises an observation window configuring an observation optical system, an illumination window configuring an illumination optical system, and an air/water supply nozzle for ejecting fluid at least to a surface of the observation window;
the air/water supply nozzle is arranged outside an ultrasound observation region possessed by the ultrasound transducer portion;
the observation window is arranged at a position outside the ultrasound observation region possessed by the ultrasound transducer portion, the position corresponding to a position where the ultrasound transducer portion is permitted to fall outside an observation field of view of the observation optical system; and
the illumination window is arranged at a position outside the ultrasound observation region possessed by the ultrasound transducer portion, the position corresponding to a position on a more outer peripheral side than the observation window.

6. The ultrasound endoscope according to claim 5, wherein
among the observation window, the illumination window and the air/water supply nozzle, the air/water supply nozzle is arranged at a position which is farthest from the ultrasound scanning plane.

7. The ultrasound endoscope according to claim 5 or 6, wherein
the observation window, the illumination window and the air/water supply nozzle are positioned so as to be substantially aligned with each other.

## Amended claims

### Amended claims under Art. 19.1 PCT

1. An ultrasound endoscope having an insertion portion to be inserted into a body cavity, the insertion portion comprising at a distal end thereof:
a distal rigid portion arranged forward of a flexible tube portion;
an ultrasound transducer portion for scanning a plane which is parallel to forward side of a longitudinal center axis of the distal rigid portion; and
a treatment instrument insertion channel port opening in a distal-side end face of the distal rigid portion, the port having a longitudinal center axis which is parallel to the longitudinal center axis of the distal rigid portion.

2. The ultrasound endoscope according to claim 1, wherein
the distal end of the insertion portion further comprises an observation optical system having an optical axis which is parallel to the longitudinal center axis of the distal rigid portion.

3. The ultrasound endoscope according to claim 1, wherein
the ultrasound transducer portion comprises a nose piece serving as a housing and an ultrasound transducer in which piezoelectric elements are arranged; and
the nose piece comprises: a tissue contact portion which is projected from the distal end surface of the distal rigid portion and has a tissue contact surface configured to coincide with the transducer lens surface; and a fixing portion which is fixed to the distal rigid portion.

4. (Amended) The ultrasound endoscope according to claim 1, wherein
the nose piece has as tissue contact portion whose outer radial dimension on a proximal end side is set so as to be approximate to a distal-end outer radius of the distal rigid portion.

5. An ultrasound endoscope having a distal rigid portion configuring an insertion portion to be inserted into a body cavity and arranged forward of a flexible
